# EUROPEAN PATENT SPECIFICATION

(11) **EP 1 676 852 B1**
(45) Date of publication and mention of the grant of the patent: **05.10.2011**
(21) Application number: 04771582.6
(22) Date of filing: 12.08.2004
(51) Int. Cl.: C07F 9/6574, C07B 57/00

(54) **PROCESS FOR PRODUCING OPTICALLY ACTIVE DIHYDROPYRIDINEPHOSPHONIC ESTER**
VERFAHREN ZUR HERSTELLUNG VON OPTISCH AKTIVEM DIHYDROPYRIDINPHOSPHONSÄUREESTER
PROCEDE DE FABRICATION D'ESTER DIHYDROPYRIDINEPHOSPHONIQUE ACTIF

(30) Priority: 01.10.2003 JP 2003342761
(43) Date of publication of application: 05.07.2006
(73) Proprietor: Nissan Chemical Industries, Ltd., Chiyoda-ku, Tokyo 101-0054 (JP)
(72) Inventor: TAKADA, Yasutaka, Nissan Chemical Industries, Ltd., Funabashi-shi Chiba 2748507 (JP); MATSUMOTO, Hiroo, Nissan Chemical Industries, Ltd., Funabashi-shi, Chiba 2748507 (JP)
(74) Representative: HOFFMANN EITLE
(86) International application number: PCT/JP2004/011607
(87) International publication number: WO 2005/033117

(56) References cited:
- SAKODA R. ET AL.: 'Synthesis and crystal structure of optically active 2-[benzyl(phenyl)amino]ethyl 5-(5,5-dimethyl-2-oxo-1,3,2-dioxaphosphorin an-2-yl)-1,4-dihydro-2,6-dimethyl-4-(3-nitr ophenyl)-3-pyridinecarboxylate (NZ-105)' CHEMICAL & PHARMACEUTICAL BULLETIN vol. 40, no. 9, 1992, pages 2377 - 2381, XP002982977
- ERNEST L ELIEL and SAMUEL H. WILEN: "Stereochemistry ofOrganic Compounds", 1994, JOHN WILEY & SONS, INC pages 158-163-298-305,

## Description

### Technical Field

The present invention relates to a process for efficiently producing an optically active substance of efonidipine (2-[Benzyl(phenyl)amino]ethyl 5-(5,5-dimethyl-2-oxo-1,3,2-dioxaphosphorinan-2-yl)-1,4-dihydro-2,6-dimethyl-4-(3-nitrophenyl)-3-pyridinecarboxylate) that is useful as an antihypertensive agent or a therapeutic agent for angina pectoris. Specifically, the present invention relates to a process for efficiently producing an optically active substance of efonidipine by preferential crystallization.

### Background Art

Efonidipine (2-[Benzyl(phenyl)amino]ethyl 5-(5,5-dimethyl-2-oxo-1,3,2-dioxaphosphorinan-2-yl)-1,4-dihydro-2,6-dimethyl-4-(3-nitrophenyl)-3-pyridinecarboxylate) is placed on the market as an antihypertensive agent or a therapeutic agent for angina pectoris, and it has been already clarified that the antihypertensive activity is attributed to one optically active substance (S(+) form) (see, for example, Patent Document 1 and Non-patent Document 1).

As a process for producing an optically active substance of efonidipine, the following three processes have been already reported:
(1) Diastereomer resolution through (2S)-2-methoxy-2-phenylethyl(4S)-5-(5,5-dimethyl-2-oxo-1,3,2-dioxaphosphorinan-2-yl)-1,4-dihydro-2,6-dimethyl-4-(3-nitrophenyl)-3-pyridine-carboxylate, shown in Fig. 1 below (see, for example, Patent Document 2 and Non-patent Document 2) 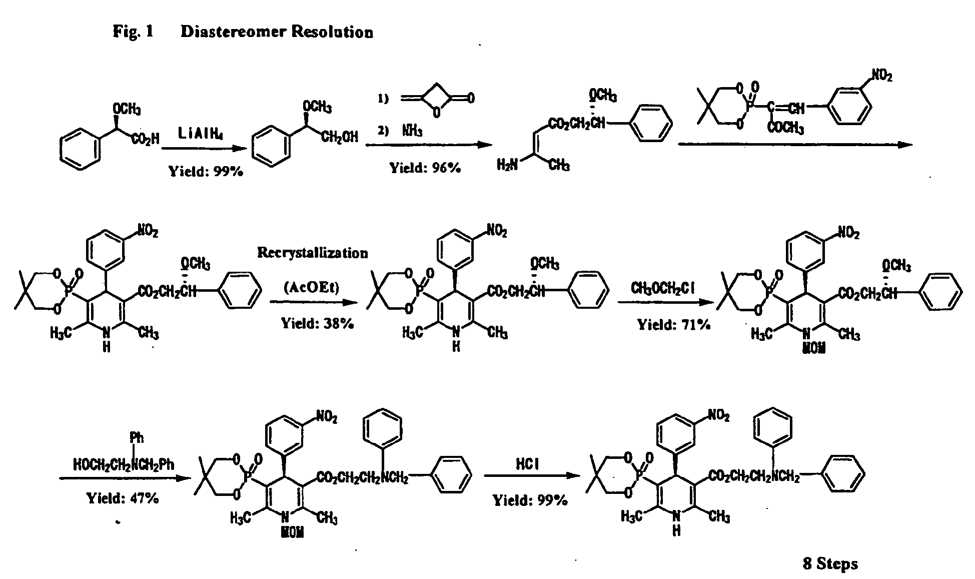
(2) Production method by utilizing enzyme shown in Fig. 2 below (see, for example, Non-patent Documents 3 and 4) 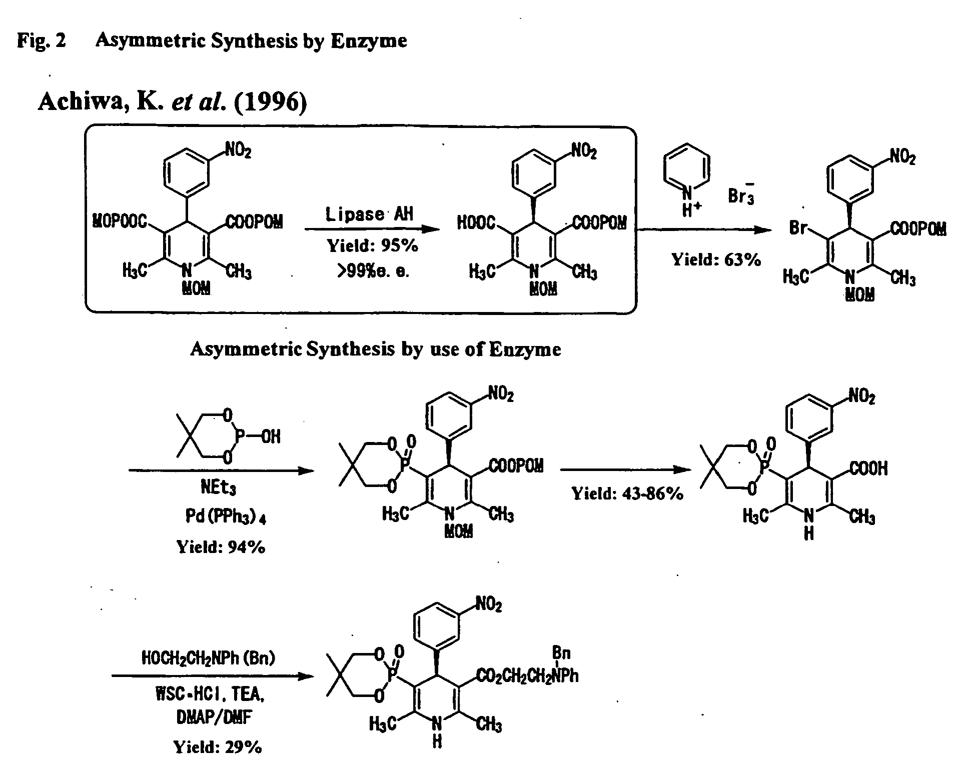
(3) Method by optical resolution with optical isomer-separation column (see, for example, Non-patent Document 5).
   Patent Document 1: JP-A-63-233992 (1988)
   Patent Document 2: JP-A-2-11592 (1990)
   Non-patent Document 1: Y. Masuda, T. Sakai, M. Sakasita, M. Takeguti, T. Takahashi, C. Arakawa, M. Hibi, S. Tanaka, K. Shigenobu, and Y. Kasuya, Jpn. J. Pharmacol., 48, p. 266 (1988)
   Non-patent Document 2: R. Sakoda, H. Matsumoto, K. Seto, Chem. Pharm. Bull., 40(9), p. 2377 (1992)
   Non-patent Document 3: T. Kato, M. Teshima, H. Ebiike, and K. Achiwa: Chem. Pharm. Bull., 44, p. 1132 (1996)
   Non-patent Document 4: H. Ebiike, Y. Yamazaki, and K. Achiwa: Chem. Pharm. Bull., 43, p. 1251 (1995)
   Non-patent Document 5: New Development of Process Chemistry (2003), p. 253, CMC Publishing Co., Ltd., Edit.: The Japanese Society of Process Chemistry

### Disclosure of Invention

### Problem to be solved by the Invention

The diastereomer resolution of (1) is inappropriate as an industrial production process as optically active alcohol used is expensive, a long time is required for steps, the yield of transesterification is low, and the introduction and removal of protective groups are required.
The production method (asymmetric synthesis) by use of enzyme of (2) is good in the yield of the step for obtaining an optically active dihydropyridine-dicarboxylate, but many steps are required for inducing the optically active substance of efonidipine, and the method has disadvantages that the yield of transesterification is low, materials are expensive and the like from the standpoint of industrial production methods.
The optical resolution by use of optical isomer-separation column of (3) is a technique that is industrially applicable, but it has problems that an expensive packing is used, a large amount of solvent is used, and a special equipment (simulated moving bed system) is required.

The problem to be solved by the present invention is to establish a process for industrially producing an optically active substance of efonidipine that is economically excellent.

### Means for solving the Problem

The present inventors eagerly investigated as to processes for efficiently producing an optically active substance of efonidipine. As a result of it, they noticed that efonidipine is a racemic mixture, found that the optically active substance can be easily produced bay carrying out preferential crystallization, and completed the present invention.

That is, the present invention relates to
1. A process for producing an optically active substance of compound of formula (1) 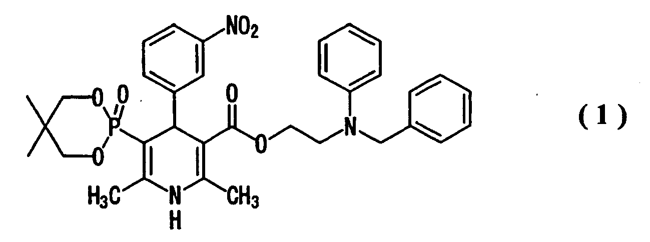 characterized by comprising:
   dissolving a racemate of the compound of formula (1) in a solvent to prepare a supersaturated solution; and
   adding crystal of either one optically active substance of the compound of formula (1)

The preferential crystallization can provide an optically active substance in a simple mode equivalent to recrystallization process of racemates if only the seed crystal is present, and is an extremely useful process from the industrial and economical standpoint.

### Best Mode for carrying out the Invention

Hereinafter, the present invention is described in further detail.

In the meantime, "n" means normal, "i" means iso, and "c" means cyclo in this specification.

Each substituent described in this specification is explained.

Halogen atom includes fluorine atom, chlorine atom, bromine atom and iodine atom. C₁₋₃alkyl group may be straight-chain, branched or cyclic, and includes for example methyl group, ethyl group, n-propyl group, i-propyl group and c-propyl group. C₁₋₃alkoxy group may be straight-chain, branched or cyclic, and includes for example methoxy group, ethoxy group, n-propoxy group, i-propoxy group and c-propoxy group.

The racemate of the compound of formula (1)used in the present invention can be easily produced according to the production method described in JP-A-63-233992 (1988). The crystal of the optically active substance of the compound of formula (1) used as a seed crystal in the present invention can be produced by use of the production method described in JP-A-2-11592 (1990), or an optical resolution with optical isomer-separation column, or the like.

The production of the optically active substance of the compound of formula (1) according to the present invention is achieved by adding crystal of either one optically active substance of the compound of formula (1) as a seed crystal in a supersaturated solution of racemate of the compound of formula (1), and allowing crystal to separate out.

The supersaturated solution can be prepared according to any conventional method such as a method comprising dissolving with heat a racemate of the compound of the formula (1) or a mixture in which either one optically active substance is present in excess in a suitable solvent, and then cooling the resulting solution, or a method comprising concentrating the solution, or a method comprising adding a solvent that lowers solubility in the solution, or the like.

The solvents used in the process for producing the optically active substance of the compound of the formula (1) include alcohols such as methyl alcohol, ethyl alcohol, 1-propanol and 2-propanol, or the like, and esters such as ethyl acetate or the like and mixed solvents in which two or more of the above-mentioned solvents are contained in an arbitrary proportion. Preferable solvents include alcohols. Preferable concrete solvents include for example methyl alcohol, ethyl alcohol, 2-propanol and ethyl acetate, more preferable solvents include for example methyl alcohol and ethyl alcohol, and further preferably it includes methyl alcohol.

The amount of the solvent used is not specifically limited so long as it is an amount range in which a racemate of the compound of formula (1) or a mixture in which either one optically active substance is present in excess becomes supersaturation. However, it is preferable to carry out in a suitable range because in case where the used amount is too much, the resulting crystal is lowered in amount, and in case where the used amount is too small, there is fear that the optical purity of the resulting crystal is lowered.

As the solubility of the compound of formula (1) differs from each other depending on the kind of the solvents, suitable used amount of the solvents cannot be generally determined. However, for example, when alcohols or esters are used as solvent, the used amount of the solvent is preferably 10 to 50 times by mass, more preferably 15 to 30 times by mass based on the used amount of the racemate of the compound of formula (1) or the mixture in which either one optically active substance is present in excess.

The added amount and grain size of the crystal of the optically active substance of the compound of formula (1) that is used as seed crystal are not specifically limited. However, it is used in an amount of 0.1 to 20 mass%, preferably 0.5 to 5 mass% based on the racemate of the compound of formula (1) or the mixture in which either one optically active substance is present in excess, in a form of ground powder.

The temperature on preparing the supersaturated solution by dissolution with heat is not specifically limited and may range from room temperature to a boiling point of the solvent. However, it is required to be controlled so that a stably supersaturated solution could be obtained depending on the solubility of the compound of formula (1) in the used solvent. For example, in case where alcohols or esters are used as a solvent, the temperature ranging from 50°C to the boiling point of the solvent is often used as a temperature on preparing the supersaturated solution by dissolution with heat.

The temperature on adding the crystal of the optically active substance of the compound of formula (1) as a seed crystal is not generally determined as it varies depending on the kind and used amount of the solvent used. However, for example, in case where alcohols or esters are used as a solvent, the temperature ranging from 40°C to 60°C is preferably used as a temperature on adding the crystal of the optically active substance.

The temperature on allowing crystal to separate out is not generally determined as it varies depending on the kind and used amount of the solvent used. However, for example, in case where alcohols or esters are used as a solvent, the temperature ranging from 20°C to 40°C is preferably used as a temperature on allowing crystal to separate out.

The time required for allowing crystal to separate out is not specifically limited so long as optical purity can be secured to some extent, but about 30 minutes to about 5 hours are sufficient therefor. In addition, the methods for allowing crystal to separate out include a method by permitting to stand or a method by carrying out with stirring, etc., but it is preferable to be carried out with stirring.

In industrial production of the optically active substance of the compound of formula (1), the following several methods that are known as preferential crystallization can be used: a batch method in which one optically active substance and the other optically active substance are resolved alternately, a continuous method in which a supersaturated solution is continuously flowed through a column in which a seed crystal is present, or a method in which one optically active substance and the other optically active substance are immersed as seed crystals in places separated by a distance and each seed crystal is grown, or the like.
When the optically active substance obtained as mentioned above is insufficient in optical purity, the optical purity can be further increased by recrystallization or the like. In this case, as an optically active substance present in excess in the mother liquor after recrystallization is different from the crystallized optically active substance, it is said to be an extremely efficient method from the standpoint of the efficiency of purification.

### Examples

Hereinafter, the present invention will be concretely described based on examples to which the present invention is not limited.
In the meantime, an optically active substance of the compound of formula (1) used in the examples was prepared according to Example 25 of JP-A-63-233992 (1988) as follows: a racemate of compound of formula (1) was synthesized and then the optionally active substance was collected with HPLC through an optical isomer-separation column.

### HPLC collection condition

Column: CHIRALCELOC (manufactured by Daicel Chemical Industries, Ltd.);
Column Size: 20 cmφ x 50 cmφ
Eluent: Methanol
Column Temperature: Room temperature
Flow Rate: 760 mL/min.
In addition, the optical purity of the optically active substance of the compound of formula (1) obtained in each example was analyzed in a condition of eluent: methanol, temperature: 40°C, flow rate: 1.0 mL/min., and UV detection: 254 nm by use of an optical isomer-separation column (CHIRALCELOC, f4.6 x 250 mm).

### Example 1 (Solvent: methanol)

In 25.0 g of methanol, 1.00 g of a racemate of compound of formula (1) was dissolved at 62°C, and then the resulting solution was cooled to 53°C over 20 minutes. The solution was seeded with 10 mg of an optically active substance of compound of formula (1) ((+) form: 100%ee), cooled to 33°C over 45 minutes, and then stirred at 30-33°C for 1 hour. After crystal separated out was collected through filtration, it was dried under reduced pressure at 50°C to obtain 144.8 mg of optically active substance ((+) form) of compound of formula (1) having an optical purity of 88.37%ee as pale yellow crystal. On the other hand, the filtrate was concentrated to obtain 771.6 mg of optically active substance ((-) form) of compound of formula (1) having an optical purity of 13.33%ee as yellow foamy substance.

### Example 2 (Solvent: ethanol)

In 25.0 g of ethanol, 1.00 g of a racemate of compound of formula (1) was dissolved at 73°C, and then the resulting solution was cooled to 53°C over 20 minutes. The solution was seeded with 10 mg of an optically active substance of compound of formula (1) ((+) form: 100%ee), cooled to 35°C over 45 minutes, and then stirred at 34-35°C for 1 hour. After crystal separated out was collected through filtration, it was dried under reduced pressure at 50°C to obtain 414.3 mg of optically active substance ((+) form) of compound of formula (1) having an optical purity of 11.42%ee as pale yellow crystal. On the other hand, the filtrate was concentrated to obtain 571.4 mg of optically active substance ((-) form) of compound of formula (1) having an optical purity of 4.35%ee as yellow foamy substance.

### Example 3 (Solvent: 1-propanol)

In 25.0 g of 1-propanol, 1.00 g of a racemate of compound of formula (1) was dissolved at 87°C, and then the resulting solution was cooled to 47°C over 55 minutes. The solution was seeded with 10 mg of an optically active substance of compound of formula (1) ((+) form: 100%ee), cooled to 30°C over 50 minutes, and then stirred at the same temperature for 1 hour. After crystal separated out was collected through filtration, it was dried under reduced pressure at 50°C to obtain 209.9 mg of optically active substance ((+) form) of compound of formula (1) having an optical purity of 52.49%ee as pale yellow crystal. On the other hand, the filtrate was concentrated to obtain 790 mg of optically active substance ((-) form) of compound of formula (1) having an optical purity of 15.45%ee as yellow foamy substance.

### Example 4 (Solvent: ethyl acetate)

In 30.0 g of ethyl acetate, 1.00 g of a racemate of compound of formula (1) was dissolved at 73°C, and then the resulting solution was cooled to 42°C over 20 minutes. The solution was seeded with 10 mg of an optically active substance of compound of formula (1) ((+) form: 100%ee), cooled to 20°C over 40 minutes, and then stirred at the same temperature for 40 minutes. After crystal separated out was collected through filtration, it was dried under reduced pressure at 50°C to obtain 87.4 mg of optically active substance ((+) form) of compound of formula (1) having an optical purity of 93.00%ee as pale yellow crystal. On the other hand, the filtrate was concentrated to obtain 920 mg of optically active substance ((-) form) of compound of formula (1) having an optical purity of 9.14%ee as yellow foamy substance. Example 5 (Repeated preferential crystallization)

In 125 g of MeOH, 5.00 g of a racemate of compound of formula (1) was dissolved at 62°C, and then the resulting solution was cooled to 51 °C over 30 minutes. The solution was seeded with 50 mg of an optically active substance of compound of formula (1) ((+) form: 100%ee), cooled to 30°C over 80 minutes, and then stirred at the same temperature for 60 minutes. After crystal separated out was collected through filtration, it was dried under reduced pressure at 50°C to obtain 0.568 g of optically active substance ((+) form) of compound of formula (1) having an optical purity of 89.97%ee as pale yellow crystal. In addition, optically active substance ((-) form) of compound of formula (1) having an optical purity of 12.04%ee was present in the filtrate. In the filtrate, 0.602 g of a racemate of compound of formula (1) was added, dissolved with heat and cooled to 50°C over 50 minutes. The solution was seeded with 50 mg of an optically active substance of compound of formula (1) ((-) form: 100%ee), cooled to 30°C over 75 minutes, and then stirred at the same temperature for 60 minutes. After crystal separated out was collected through filtration, it was dried under reduced pressure at 50°C to obtain 1.364 g of optically active substance ((-) form) of compound of formula (1) having an optical purity of 86.92%ee as pale yellow crystal. In addition, optically active substance ((+) form) of compound of formula (1) having an optical purity of 17.87%ee was present in the filtrate. In the filtrate, 1.360 g of a racemate of compound of formula (1) and 15.0 g of methanol were added, dissolved with heat again and cooled to 54°C over 35 minutes. The solution was seeded with 50 mg of an optically active substance of compound of formula (1) ((+) form: 100%ee), cooled to 30°C over 55 minutes, and then stirred at the same temperature for 55 minutes. After crystal separated out was collected through filtration, it was dried under reduced pressure at 50°C to obtain 1.270 g of optically active substance ((+) form) of compound of formula (1) having an optical purity of 93.05%ee as pale yellow crystal. In addition, optically active substance ((-) form) of compound of formula (1) having an optical purity of 14.23%ee was present in the filtrate.

### Reference Example 6 (Purification by recrystallization, Case 1)

A mixture of 899.6 mg of an optically active substance of compound of formula (1) ((+) form: 100%ee) with 100.2 mg of a racemate of compound of formula (1) was dissolved in 25.0 g of ethanol at 77°C. An optical purity was 88.80%ee at this point. The resulting solution was cooled to 28°C over 35 minutes, and then cooled further to 23°C over 45 minutes. After crystal separated out was collected through filtration, it was dried under reduced pressure at 50°C to obtain 891 mg of optically active substance ((+) form) of compound of formula (1) having an optical purity of 99.71%ee as pale yellow crystal. On the other hand, the filtrate was concentrated to obtain 0.10 g of optically active substance ((-) form) of compound of formula (1) having an optical purity of 7.42%ee as yellow foamy substance.

### Reference Example 7 (Purification by recrystallization, Case 2)

A mixture of 80.6 mg of an optically active substance of compound of formula (1) ((+) form: 100%ee) with 420.4 mg of a racemate of compound of formula (1) was dissolved in 12.5 g of ethanol at 76°C. An optical purity was 16.70%ee at this point. The resulting solution was cooled to a temperature of 5°C or below over 40 minutes, and stirred at the same temperature for 30 minutes. After crystal separated out was collected through filtration, it was dried under reduced pressure at 50°C to obtain 371.7 mg of optically active substance ((+) form) of compound of formula (1) having an optical purity of 26.01 %ee as yellow solid. In addition, the filtrate was concentrated to obtain 0.12 g of optically active substance ((-) form) of compound of formula (1) having an optical purity of 7.55%ee as yellow foamy substance.

### Reference Example 8 (Purification by toluene crystallization)

A mixture of 51.7 mg of an optically active substance of compound of formula (1) ((+) form: 100%ee) with 449.0 mg of a racemate of compound of formula (1) was dissolved in 7.50 g of toluene at 95°C. An optical purity was 10.20%ee at this point. The resulting solution was cooled to 60°C over 7 minutes. Thereafter, the solution was seeded with 5.2 mg of toluene solvate of a racemate of compound of formula (1), cooled to a temperature of 5°C or below over 40 minutes and stirred at the same temperature for 30 minutes. After crystal separated out was collected through filtration, it was dried under reduced pressure at 60°C to obtain 506.8 mg of toluene solvate of a racemate of compound of formula (1) (as actually an optically active substance ((+) form) of compound of formula (1) was in slightly excess, an optical purity was 5.65%ee) as yellow solid. In addition, the filtrate was concentrated to obtain 0.05 g of optically active substance ((+) form) of compound of formula (1) having an optical purity of 97.96%ee as yellow residue.

### Reference Example 9 (Preferential crystallization in the presence of methanesulfonic acid)

In 3.00 (4.75 mmol) g of a racemate of compound of formula (1), 300 mg (3.12 mmol, 0.66 equivalent) of methanesulfonic acid was added, the resulting solution was refluxed with heat (62°C) in 15.0 g of methanol, and dissolved. The resulting solution was cooled to 50°C over 30 minutes, and then 10 mg of an optically active substance of compound of formula (1) ((+) form: 100%ee) was added, cooled to 40°C over 1 hour, and then stirred at the same temperature for 1 hour. After crystal separated out was collected through filtration, it was washed with 2 ml of methanol and dried under reduced pressure at 50°C to obtain 716.2 mg of optically active substance ((+) form) of compound of formula (1) having an optical purity of 30.62%ee as white solid. On the other hand, optically active substance ((-) form) of compound of formula (1) present in 14.42 g of the filtrate had 3.82%ee.

### Industrial Applicability

The present invention establishes a process for producing an optically active efonidipine that is economically excellent.

## Claims

1. A process for producing an optically active substance of compound of formula (1) 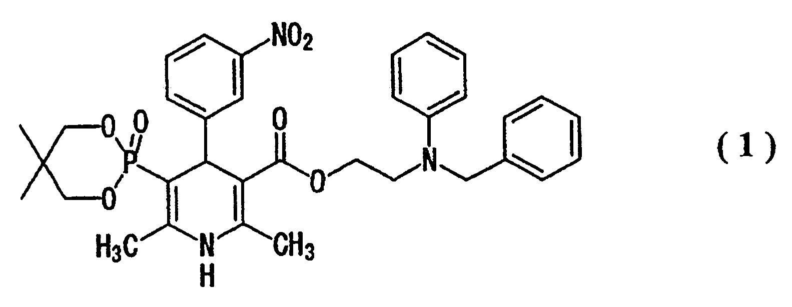 **characterized by** comprising:
dissolving a racemate of the compound of formula (1) in a solvent, which is alcohols or esters, to prepare a supersaturated solution; and
adding a crystal of either one optically active substance of the compound of formula (1) as a seed crystal in the supersaturated solution to allow crystal of the one optically active substance added as the seed crystal to separate out.

2. The process according to claim 1, wherein the solvent is methyl alcohol.

## Patentansprüche

1. Verfahren zur Erzeugung einer optisch aktiven Substanz einer Verbindung der Formel (1): 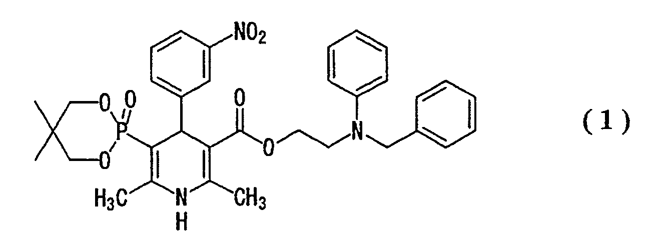 **gekennzeichnet durch**:
Auflösen eines Razemats der Verbindung der Formel (1) in einem Lösungsmittel, das ein Alkohol oder Ester ist, zur Herstellung einer supergesättigten Lösung, und
Zugabe eines Kristalls von einer der optisch aktiven Substanzen der Verbindung der Formel (1) als Keimkristall zu der supergesättigten Lösung, um zu ermöglichen, dass der Kristall der einen optisch aktiven Substanz, die als Keimkristall zugegeben ist, sich abtrennt.

2. Verfahren nach Anspruch 1, worin das Lösungsmittel Methylalkohol ist.

## Revendications

1. Procédé pour produire une substance optiquement active du composé de formule (1) 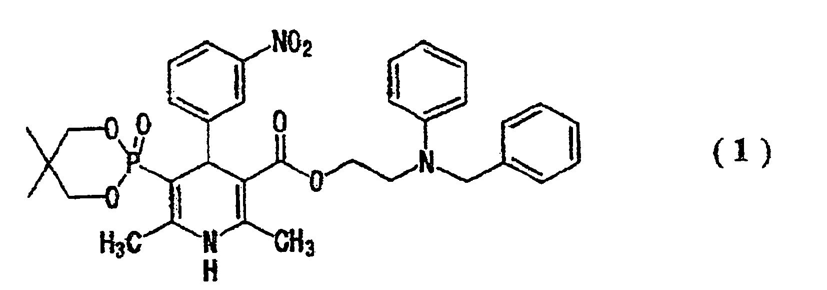 **caractérisé en ce qu'**il comprend :
la dissolution d'un racémate du composé de formule (1) dans un solvant, qui est des alcools ou des esters, pour préparer une solution sursaturée ; et
l'addition d'un cristal de l'une ou l'autre substance optiquement active du composé de formule (1) comme germe cristallin dans la solution sursaturée pour permettre à un cristal de la substance optiquement active ajoutée comme germe cristallin de se séparer.

2. Procédé selon la revendication 1 où le solvant est l'alcool méthylique.
